# EUROPEAN PATENT APPLICATION

(11) **EP 2 314 569 A1**
(43) Date of publication of application: **27.04.2011**
(21) Application number: 09013361.2
(22) Date of filing: 22.10.2009
(51) Int. Cl.: C07C 237/06, A61K 31/165, A61P 25/00

(54) **Novel polymorphic forms of (S)-2-[4-(3-Fluoro-benzyloxy)-benzylamino]-propionamide mesylate salt and processes of manufacturing thereof**

(71) Applicant: Merck Patent GmbH, 64293 Darmstadt (DE)
(72) Inventor: Schlueter, Doris, Dr., 64291 Darmstadt (DE); Saal, Christoph, Dr., 64853 Otzberg (DE); Kuehn, Clemens, Dr., 64291 Darmstadt (DE); Schlueter, Tobias, 64331 Weiterstadt (DE)

(57) **Abstract**

The present invention relates to (S)-2-[4-(3-Fluoro-benzyloxy)-benzylamino]-propionamide mesylate anhydrate and crystalline modifications thereof. The present invention further relates to processes of manufacturing these crystalline modifications as well as their use in the treatment and/or prophylaxis of physiological and/or pathophysiological conditions selected from the group consisting of "central nervous system disorders, epilepsy, Parkinson's disease, neurodegenerative processes, depression, spasm, hypnosis, analgesia, chronic pain, neuropathic pain, head pain conditions, migraine, headache, cluster headache, neuralgia, hemicrania, facial pain, arachnoiditis, severe headache, restless legs syndrome, addictive disorders, motor restlessness, creeping, burning, pulling sensations, drug abuse, severe alcoholism, reward deficiency syndrome, cognitive disorders, autism, dyslexia, attention deficit hyperactivity disorder, anxiety, schizophrenia, obsessive compulsive disorders, psychosis, bipolar disorder, Tourette's syndrome, mild cognitive impairment and disorders of learning in children, adolescents and adults, age associated memory impairment, age associated cognitive decline, Alzheimer's disease, Down's Syndrome, traumatic brain injury Huntington's disease, progressive supranuclear Palsy, HIV, stroke, vascular diseases, Pick's or Creutzfeldt-Jacob diseases, multiple sclerosis".

## Description

### Technical field

The present invention relates to (S)-2-[4-(3-Fluoro-benzyloxy)-benzylamino]-propionamide mesylate anhydrate and crystalline modifications thereof as well as their medical uses and processes of manufacturing.

### Prior art

### (S)-2-[4-(3-Fluoro-benzyloxy)-benzylamino]-propionamide (1)

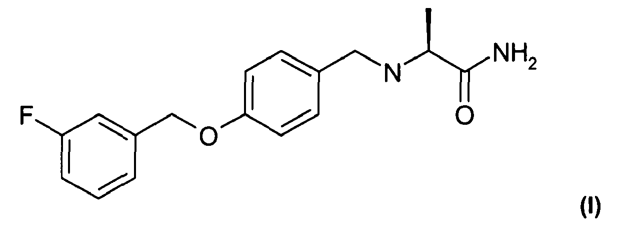

was first described in WO 90/14334 and disclosed as free base. However, pharmaceutically acceptable salt formation with methanesulfonic acid as one of many organic acids is also mentioned. The compounds described in WO 90/14334 were found to act on the central nervous system, e.g. as anti-epileptic, anti-Parkinson, neuroprotective, antidepressant, antispastic and/or hypnotic agents. No solvates or crystalline modifications are disclosed.

Pevarello et al. (J. Med. Chem. 1998, 41: 579-590) describe the synthesis of (S)-2-[4-(3-Fluoro-benzyloxy)-benzylamino]-propionamide methanesulfonate (mesylate) as an anticonvulsant agent (compound 57). No description of the synthesis of the mesylate salt is given. The only information referring to the solid state form is given from the melting point (210°C).

WO 99/35125 describes the use of (S)-2-[4-(3-Fluoro-benzyloxy)-benzylamino]-propionamide methanesulfonate (mesylate) as analgesic agent, for instance for the treatment or alleviation of chronic or neuropathic pain. No solvates or crystalline modifications are disclosed.

WO 2004/062655 discloses (S)-2-[4-(3-Fluoro-benzyloxy)-benzylamino]-propionamide as free base. However, pharmaceutically acceptable salt formation with methanesulfonic acid as one of many organic acids is also mentioned. The compounds described in WO 2004/062655 were found to act as anti-migraine agents, particularly for the treatment of head pain conditions such as migraine, headache, cluster headache, neuralgia, hemicrania, facial pain, arachnoiditis or other severe headache. No solvates or crystalline modifications are disclosed.

WO 2004/089353 describes the combinational use of safinamide and a Parkinson's disease agent for the treatment of Parkinson's disease. Pharmaceutically acceptable salt formation with methanesulfonic acid as one of many organic acids is also mentioned. No solvates or crystalline modifications are disclosed.

WO 2005/102300 discloses the use of (S)-2-[4-(3-Fluoro-benzyloxy)-benzylamino]-propionamide as free base. However, pharmaceutically acceptable salt formation with methanesulfonic acid as one of many organic acids is also mentioned. The compounds described in WO 2005/102300 were found to be useful for the treatment of restless legs syndrome and addictive disorders, such as motor restlessness, creeping, burning, pulling sensations, drug abuse, severe alcoholism and reward deficiency syndrome. No solvates or crystalline modifications are disclosed.

Binda C et al. (J. Med. Chem. 2007, 50: 5848-5852) is directed to structures of human monoamine oxidase B complexes with selective noncovalent inhibitors safinamide and coumarin analogs. However, no solvates or crystalline modifications of safinamide itself are disclosed.

WO 2007/144153 describes the use of safinamide in the treatment of cognitive disorders, in particular autism, dyslexia, attention deficit hyperactivity disorder, anxiety, schizophrenia, obsessive compulsive disorders, psychosis, bipolar disorders, depression, Tourette's syndrome, Mild Cognitive Impairment and disorders of learning in children, adolescents and adults, Age Associated Memory Impairment, Age Associated Cognitive Decline, Alzheimer's Disease, Parkinson's Disease, Down's Syndrome, traumatic brain injury Huntington's Disease, Progressive Supranuclear Palsy, HIV, stroke, vascular diseases, Pick's or Creutzfeldt-Jacob diseases and multiple sclerosis. Pharmaceutically acceptable salt formation with methanesulfonic acid as one of many organic acids is also mentioned. No solvates or crystalline modifications are disclosed.

WO 2007/147491 discloses a process of the production of safinamide. Safinamide (example 6) and safinamide methanesulfonate (example 7) are prepared and dried in vacuo. The drying step yielded crystalline safinamide anhydrate in its crystalline modification A1.

WO 2009/074478 describes another process for the preparation of safinamide. Safinamide (examples 12a, 12a1, 16b and 20) and safinamide methanesulfonate (examples 12b, 12b1, 13, 16c and 20) are prepared and dried in vacuo. The drying step yielded crystalline safinamide anhydrate in its crystalline modification A1.

Certain crystalline, i.e. morphological or polymorphic forms of pharmaceutical compounds may be of interest to those involved in the development of suitable pharmaceutical dosage forms. This is because if a certain polymorphic form is not held constant during clinical and stability studies, the exact dosage used or measured may not be comparable from one batch to the other. Once a pharmaceutical compound is produced for use, it is important to verify the morphological or polymorphic form delivered in the active pharmaceutical ingredient and in each dosage form to assure that production processes for drug substance and drug product deliver the same form . Therefore, it is imperative to assure that either a single morphological or polymorphic form or a known combination of morphological or polymorphic forms is present. In addition, certain morphological or polymorphic forms may exhibit enhanced thermodynamic stability or other advantages and may be more suitable than other morphological or polymorphic forms for inclusion in pharmaceutical formulations. Regulatory guidance for addressing polymorphism for pharmaceutical applications for drug substances and drug products can be found in "ICH Harmonised Tripartite Guideline ― Specifications: Test Procedures and Acceptance Criteria for New Drug Substances and New Drug Products: Chemical Substances Q6A", 6^{th} of October 1999, http://www.ich.org.

The citation of any reference in this application is not an admission that the reference is relevant prior art to this application.

### Description of the invention

The present invention has the object to provide novel polymorphic forms of (S)-2-[4-(3-Fluoro-benzyloxy)-benzylamino]-propionamide mesylate anhydrate.

The object of the present invention has surprisingly been solved in one aspect by providing (S)-2-[4-(3-Fluoro-benzyloxy)-benzylamino]-propionamide mesylate anhydrate.

It should be understood that the present anhydrates of the invention may contain unbound or physisorbed water that is to say water which is other than water of crystallization and conclusively not part of the crystal lattice.

Further, the molar ratio of methanesulfonic acid to (S)-2-[4-(3-Fluoro-benzyloxy)-benzylamino]-propionamide (free base) within (S)-2-[4-(3-Fluoro-benzyloxy)-benzylamino]-propionamide mesylate can vary for each and all the herein disclosed anhydrates and their crystalline modifications as known to persons skilled in the art. Preferably, the molar ratio is between 0.5:1 to 1.5:1, more preferably between 0.8:1 to 1.2:1, most preferably 1:1.

The object of the present invention has surprisingly been solved in another aspect by providing (S)-2-[4-(3-Fluoro-benzyloxy)-benzylamino]-propionamide mesylate anhydrate in its crystalline modification NF6, which is characterized by XRD peaks comprising 17.9°, 18.8°, 19.6°, 20.7°, and 23.0° (in °2θ using Cu-Kα₁ radiation, ± 0.1° at -5 °C).

The object of the present invention has surprisingly been solved in another aspect by providing (S)-2-[4-(3-Fluoro-benzyloxy)-benzylamino]-propionamide mesylate anhydrate in its crystalline modification NF6, which is characterized by XRD peaks comprising 7.9°, 17.9°, 18.8°, 19.6°, 20.1 °, 20.7, 21.2°, 23.0°, 23.5° and 24.8° (in °2θ using Cu-Kα₁ radiation, ± 0.1° at -5 °C).

The object of the present invention has surprisingly been solved in another aspect by providing (S)-2-[4-(3-Fluoro-benzyloxy)-benzylamino]-propionamide mesylate anhydrate in its crystalline modification NF6, which is characterized by the following XRD data:

**Form NF6:**

| **Peak No.** | **d/Å at -5 ºC** | **°2**θ **at -5 °C (Cu-K**α**₁ radiation)** | **h** | **k** | **l** |
|---|---|---|---|---|---|
| 1 | 20.1 | 4.4 | 0 | 1 | 1 |
| 2 | 11.2 | 7.9 | 0 | 2 | 0 |
| 3 | 7.3 | 12.1 | 0 | 1 | 6 |
| 4 | 5.6 | 15.9 | 0 | 4 | 0 |
| 5 | 5.0 | 17.9 | 1 | 2 | 0 |
| 6 | 4.7 | 18.8 | 1 | 2 | 3 |
| 7 | 4.5 | 19.6 | 0 | 4 | 6 |
| 8 | 4.5 | 19.7 | 1 | 0 | 6 |
| 9 | 4.4 | 20.1 | 1 | 1 | 6 |
| 10 | 4.3 | 20.7 | 0 | 5 | 3 |
| 11 | 4.3 | 20.8 | 1 | 3 | 3 |
| 12 | 4.2 | 21.2 | 1 | 2 | 6 |
| 13 | 3.9 | 23.0 | 0 | 5 | 6 |
| 14 | 3.9 | 23.0 | 1 | 3 | 6 |
| 15 | 3.8 | 23.2 | 0 | 1 | 12 |
| 16 | 3.8 | 23.4 | 0 | 4 | 9 |
| 17 | 3.8 | 23.5 | 1 | 0 | 9 |
| 18 | 3.6 | 24.8 | 1 | 2 | 9 |
| 19 | 3.2 | 28.1 | 1 | 5 | 6 |

Crystalline modification NF6 is further characterized by the following parameter: orthorhombic, space group P2₁2₁2₁
lattice constants (at -5 °C):
a = 5.5Å,
b = 22.3 Å
c **=** 46.6Å
cell volume = 5771 Å³

In the course of the present invention, the term "crystalline modification" is used as a synonym for terms "crystalline form", "polymorphic form", "polymorphic modification", "morphological form" and the like.

The crystalline modifications of the present invention, in particular NF6, are surprisingly characterized by, among others, a reduced hygroscopicity, a better compressibility during the tableting process, a prolonged shelf life, a better thermodynamic stability, i.e. stability against heat and humidity, a better resistance to sunlight, i.e. UV-light, an increased bulk density, an improved solubility, bioavailability characteristics which are constant from one batch to the other, better flow and handling properties in the tableting process, an improved colour stability and better filtration properties in the production process. Therefore, by use of the crystalline modifications of the present invention, it is possible to obtain pharmaceutical formulations with improved processability, homogeneity, stability, purity and uniformity from one batch to the other.

Furthermore, crystalline modification NF6 of the present invention is surprisingly better suited for (pharmaceutical) formulations that have to be stored or that are processed at low temperatures (e.g. cooling crystallization from non-aqueous solvents).

The crystalline modifications of the present invention can be characterized according to standard methods which can be found e.g. in Rolf Hilfiker, 'Polymorphism in the Pharmaceutical Industry', Wiley-VCH, Weinheim 2006, and references therein, e.g. X-Ray diffraction (XRD; chapter 6), lR and Raman spectroscopy (chapter 5), Differential Scanning Calorimetry (DSC) and Thermogravimetric Analysis (TGA) (chaper 3), Water Vapour Sorption Studies (chapter 9), or which can be found e.g. in H.G. Brittain (editor), Polymorphism in Pharmaceutical Solids, Vol. 95, Marcel Dekker Inc., New York 1999 (chapter 6: all there mentioned techniques).

(S)-2-[4-(3-Fluoro-benzyloxy)-benzylamino]-propionamide mesylate anhydrate and (S)-2-[4-(3-Fluoro-benzyloxy)-benzylamino]-propionamide mesylate anhydrate in its crystalline modification NF6 is hereinafter referred to as "product(s) of the (present) invention".

In another aspect of the invention, a pharmaceutical composition comprising a therapeutically effective amount of at least one product of the invention is provided.

In a preferred embodiment, the pharmaceutical composition comprises 1% to 50% (S)-2-[4-(3-Fluoro-benzyloxy)-benzylamino]-propionamide mesylate anhydrate in its crystalline modification NF6 as described herein and 1% to 50% (S)-2-[4-(3-Fluorobenzyloxy)-benzylamino]-propionamide mesylate anhydrate in its crystalline modification A1, which is characterized by XRD peaks comprising 18.6°, 20.3°, 20.6° and 22.8° (in °2θ using Cu-Kα₁ radiation, ± 0.1°), preferably characterized by XRD peaks comprising 7.8°, 12.0°, 15.6°, 17.7°, 18.6°, 19.3°, 20.3°, 20.6°, 22.8° and 29.0° (in °2θ using Cu-Kα₁radiation, ± 0.1°), and even more preferably characterized by the following XRD data:

**Form A1:**

| **Peak No.** | **d/Å** | **°2**θ **(Cu-K**α**₁ radiation)** ±**0.1°** | **h** | **k** | **l** |
|---|---|---|---|---|---|
| 1 | 11.4 | 7.8 | 0 | 0 | 2 |
| 2 | 9.2 | 9.6 | 0 | 1 | 2 |
| 3 | 7.4 | 12.0 | 0 | 2 | 1 |
| 4 | 6.8 | 13.0 | 0 | 1 | 3 |
| 5 | 5.7 | 15.6 | 0 | 0 | 4 |
| 6 | 5.1 | 17.4 | 1 | 1 | 1 |
| 7 | 5.0 | 17.7 | 1 | 0 | 2 |
| 8 | 4.8 | 18.6 | 1 | 1 | 2 |
| 9 | 4.6 | 19.3 | 0 | 2 | 4 |
| 10 | 4.4 | 20.3 | 0 | 1 | 5 |
| 11 | 4.3 | 20.6 | 1 | 1 | 3 |
| 12 | 3.9 | 22.8 | 1 | 2 | 3 |
| 13 | 3.1 | 29.0 | 1 | 1 | 6 |

In a preferred embodiment, the pharmaceutical composition further comprises at least one additional compound selected from the group consisting of physiologically acceptable excipients, auxiliaries, adjuvants, diluents, carriers and/or additional pharmaceutically active substances other than the products of the invention.

A further embodiment of the present invention is a process for the manufacture of said pharmaceutical compositions, characterized in that one or more products of the invention and one or more compounds selected from the group consisting of solid, liquid or semiliquid excipients, auxiliaries, adjuvants, diluents, carriers and pharmaceutically active substances other than the products of the invention, are converted in a suitable dosage form.

As used herein, the term "effective amount" refers to any amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

In another aspect of the invention, the use of a product of the invention or a pharmaceutical composition as described herein as a dopamine modulator, monoamine oxidase B inhibitor and/or dopamine reuptake inhibitor is provided.

In another aspect of the invention, a medicament comprising at least one product of the invention or a pharmaceutical composition as described herein is provided.

In a further aspect of the invention, a medicament as described herein for use in the treatment and/or prophylaxis of physiological and/or pathophysiological conditions selected from the group consisting of: "central nervous system disorders, epilepsy, Parkinson's disease, neurodegenerative processes, depression, spasm, hypnosis, analgesia, chronic pain, neuropathic pain, head pain conditions, migraine, headache, cluster headache, neuralgia, hemicrania, facial pain, arachnoiditis, severe headache, restless legs syndrome, addictive disorders, motor restlessness, creeping, burning, pulling sensations, drug abuse, severe alcoholism, reward deficiency syndrome, cognitive disorders, autism, dyslexia, attention deficit hyperactivity disorder, anxiety, schizophrenia, obsessive compulsive disorders, psychosis, bipolar disorder, Tourette's syndrome, mild cognitive impairment and disorders of learning in children, adolescents and adults, age associated memory impairment, age associated cognitive decline, Alzheimer's disease, Down's Syndrome, traumatic brain injury Huntington's disease, progressive supranuclear Palsy, HIV, stroke, vascular diseases, Pick's or Creutzfeldt-Jacob diseases, multiple sclerosis" is provided. A corresponding use for the preparation of a medicament for the treatment and/or prophylaxis of the aforementioned conditions is intended to be comprised.

ln another aspect of the invention, a medicament as described herein is provided, wherein in such medicament comprises at least one additional pharmacologically active substance (drug, ingredient).

In another aspect of the invention, a medicament as described herein is provided, wherein the medicament is applied before and/or during and/or after treatment with at least one additional pharmacologically active substance.

In a further aspect of the invention, a kit comprising a therapeutically effective amount of at least one product of the invention and/or at least one pharmaceutical composition as described herein and a therapeutically effective amount of at least one further pharmacologically active substance other than the products of the invention is provided.

Products of the invention may be used in combination with one or more other pharmacologically active substances (ingredients, drugs) in the treatment, prevention, suppression or amelioration of diseases or conditions for which products of the invention or the other substances have utility. Typically the combination of the drugs is safer or more effective than either drug alone, or the combination is safer or more effective than would it be expected based on the additive properties of the individual drugs. Such other drug(s) may be administered, by a route and in an amount commonly used contemporaneously or sequentially with a product of the invention. When a product of the invention is used contemporaneously with one or more other drugs, a combination product containing such other drug(s) and the product of the invention is preferred. However, combination therapy also includes therapies in which the product of the invention and one or more other drugs are administered on different overlapping schedules. It is contemplated that when used in combination with other active ingredients, the product of the present invention or the other active ingredient or both may be used effectively in lower doses than when each is used alone. Accordingly, the pharmaceutical compositions of the present invention (pharmaceutical compositions as described herein) include those that contain one or more other active ingredients, in addition to a product of the invention.

The pharmaceutical compositions of the present invention (as described herein) may be administered by any means that achieve their intended purpose. For example, administration may be by oral, parenteral, topical, enteral, intravenous, intramuscular, inhalant, nasal, intraarticular, intraspinal, transtracheal, transocular, subcutaneous, intraperitoneal, transdermal, or buccal routes. Alternatively, or concurrently, administration may be by the oral route. The dosage administered will be dependent upon the age, health, and weight of the recipient, kind of concurrent treatment, if any, frequency of treatment, and the nature of the effect desired. Parenteral administration is preferred. Oral administration is especially preferred.

Suitable dosage forms include, but are not limited to capsules, tablets, pellets, dragees, semi-solids, powders, granules, suppositories, ointments, creams, lotions, inhalants, injections, cataplasms, gels, tapes, eye drops, solution, syrups, aerosols, suspension, emulsion, which can be produced according to methods known in the art, for example as described below:
tablets: mixing of active ingredient/s and auxiliaries, compression of said mixture into tablets (direct compression), optionally granulation of part of mixture before compression.
capsules: mixing of active ingredient/s and auxiliaries to obtain a flowable powder, optionally granulating powder, filling powders/granulate into opened capsules, capping of capsules.
semi-solids (ointments, gels, creams): dissolving/dispersing active ingredient/s in an aqueous or fatty carrier; subsequent mixing of aqueous/fatty phase with complementary fatty/ aqueous phase, homogenization (creams only).
suppositories (rectal and vaginal): dissolving/dispersing active ingredient/s in carrier material liquified by heat (rectal: carrier material normally a wax; vaginal: carrier normally a heated solution of a gelling agent), casting said mixture into suppository forms, annealing and withdrawal suppositories from the forms.
aerosols: dispersing/dissolving active agent/s in a propellant, bottling said mixture into an atomizer.

In general, non-chemical routes for the production of pharmaceutical compositions and/or pharmaceutical preparations comprise processing steps on suitable mechanical means known in the art that transfer one or more products of the invention into a dosage form suitable for administration to a patient in need of such a treatment. Usually, the transfer of one or more products of the invention into such a dosage form comprises the addition of one or more compounds, selected from the group consisting of carriers, excipients, auxiliaries and pharmaceutical active ingredients other than the products of the invention. Suitable processing steps include, but are not limited to combining, milling, milling at low temperature, extruding, spray-drying, embedding, mixing, granulating, freeze-drying, dissolving, dispersing, homogenizing, casting and/or compressing the respective active and non-active ingredients. Mechanical means for performing said processing steps are known in the art, for example from Ullmann's Encyclopedia of Industrial Chemistry, 5th Edition. In this respect, active ingredients are preferably at least one product of the invention and one or more additional compounds other than the products of the invention, which show valuable pharmaceutical properties, preferably those pharmaceutical active agents other than the products of the invention, which are disclosed herein.

Particularly suitable for oral use are tablets, pills, coated tablets, capsules, powders, granules, syrups, juices or drops, suitable for rectal use are suppositories, suitable for parenteral use are solutions, preferably oil-based or aqueous solutions, furthermore suspensions, emulsions or implants, and suitable for topical use are ointments, creams or powders. The products of the invention may also be lyophilised and the resultant lyophilisates used, for example, for the preparation of injection preparations. The preparations indicated may be sterilised and/or comprise assistants, such as lubricants, preservatives, stabilisers and/or wetting agents, emulsifiers, salts for modifying the osmotic pressure, buffer substances, dyes, flavours and/or a plurality of further active ingredients, for example one or more vitamins.

Suitable excipients are organic or inorganic substances, which are suitable for enteral (for example oral), parenteral or topical administration and do not react with the products of the invention, for example water, vegetable oils, benzyl alcohols, alkylene glycols, polyethylene glycols, glycerol triacetate, gelatine, carbohydrates, such as lactose, sucrose, mannitol, sorbitol or starch (maize starch, wheat starch, rice starch, potato starch), cellulose preparations and/or calcium phosphates, for example tricalcium phosphate or calcium hydrogen phosphate, magnesium stearate, talc, gelatine, tragacanth, methyl cellulose, hydroxypropylmethylcellulose, sodium carboxymethylcellulose, polyvinyl pyrrolidone and/or vaseline.

If desired, disintegrating agents may be added such as the above-mentioned starches and also carboxymethyl-starch, cross-linked polyvinyl pyrrolidone, agar, or alginic acid or a salt thereof, such as sodium alginate. Auxiliaries include, without limitation, flow-regulating agents and lubricants, for example, silica, talc, stearic acid or salts thereof, such as magnesium stearate or calcium stearate, and/or polyethylene glycol. Dragee cores are provided with suitable coatings, which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices or to provide a dosage form affording the advantage of prolonged action, the tablet, dragee or pill can comprise an inner dosage and an outer dosage component me latter being in the form of an envelope over the former. The two components can be separated by an enteric layer, which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol, solutions of suitable cellulose preparations such as acetyl-cellulose phthalate, cellulose acetate or hydroxypropylmethyl-cellulose phthalate, are used. Dye stuffs or pigments may be added to the tablets or dragee coatings, for example, for identification or in order to characterize combinations of active compound doses.

Suitable carrier substances are organic or inorganic substances which are suitable for enteral (e.g. oral) or parenteral administration or topical application and do not react with the novel compounds, for example water, vegetable oils, benzyl alcohols, polyethylene glycols, gelatine, carbohydrates such as lactose or starch, magnesium stearate, talc and petroleum jelly. In particular, tablets, coated tablets, capsules, syrups, suspensions, drops or suppositories are used for enteral administration, solutions, preferably oily or aqueous solutions, furthermore suspensions, emulsions or implants, are used for parenteral administration, and ointments, creams or powders are used for topical application. The products of the invention can also be lyophilized and the lyophilizates obtained can be used, for example, for the production of injection preparations.

The preparations indicated can be sterilized and/or can contain excipients such as lubricants, preservatives, stabilizers and/or wetting agents, emulsifiers, salts for affecting the osmotic pressure, buffer substances, colorants, flavourings and/or aromatizers. They can, if desired, also contain one or more further active compounds, e.g. one or more vitamins.

Other pharmaceutical preparations, which can be orally used include push-fit capsules made of gelatine or HPMC, as well as soft, sealed capsules made of gelatine and a plasticizer such as glycerol or sorbitol. The push-fit capsules can contain the active compounds in the form of granules, which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatine.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400).

Aqueous injection suspensions may contain substances, which increase the viscosity of the suspension, including, for example, sodium carboxymethyl cellulose, sorbitol, and/or dextran, optionally the suspension may also contain stabilizers.

For administration as an inhalation spray, it is possible to use sprays in which the active ingredient is either dissolved or suspended in a propellant gas or propellant gas mixture (for example CO₂ or chlorofluorocarbons). The active ingredient is advantageously used here in micronized form, in which case one or more additional physiologically acceptable solvents may be present, for example ethanol. Inhalation solutions can be administered with the aid of conventional inhalers.

Possible pharmaceutical preparations, which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatine rectal capsules, which consist of a combination of the active compounds with a base. Possible base materials include, for example, liquid triglycerides, polyethylene glycols, or paraffin hydrocarbons.

The pharmaceutical preparations can be employed as medicaments in human and veterinary medicine. As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function. Said therapeutic effective amount of one or more of the products of the invention is known to the skilled artisan or can be easily determined by standard methods known in the art.

The products of the invention and the additional pharmacologically active substances are generally administered analogously to commercial preparations. Usually, suitable doses that are therapeutically effective lie in the range between 0.0005 mg and 1000 mg, preferably between 0.005 mg and 500 mg and especially between 0.5 mg and 100 mg per dose unit. The daily dose is preferably between about 0.001 mg/kg and 10 mg/kg of body weight.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

For the purpose of the present invention, all mammalian species are regarded as being comprised. In a preferred embodiment, such mammals are selected from the group consisting of "primate, human, rodent, equine, bovine, canine, feline, domestic animals, cattle, livestock, pets, cow, sheep, pig, goat, horse, pony, donkey, hinny, mule, hare, rabbit, cat, dog, guinea pig, hamster, rat and mouse". More preferably, such mammals are humans. Animal models are of interest for experimental investigations, providing a model for treatment of human diseases.

The specific dose for the individual patient depends, however, on the multitude of factors, for example on the efficacy of the specific compounds employed, on the age, body weight, general state of health, the sex, the kind of diet, on the time and route of administration, on the excretion rate, the kind of administration and the dosage form to be administered, the pharmaceutical combination and severity of the particular disorder to which the therapy relates. The specific therapeutic effective dose for the individual patient can readily be determined by routine experimentation, for example by the doctor or physician, which advises or attends the therapeutic treatment.

In the case of many disorders, the susceptibility of a particular cell to treatment with the subject compounds may be determined by in vitro testing. Typically a culture of the cell is combined with a subject compound at varying concentrations for a period of time sufficient to allow the active agents to show a relevant reaction, usually between about one hour and one week. For in vitro testing, cultured cells from a biopsy sample may be used.

The object of the present invention has surprisingly been solved in another aspect by providing a process for manufacturing crystalline modification NF6 of (S)-2-[4-(3-Fluoro-benzyloxy)-benzylamino]-propionamide mesylate anhydrate as described herein, comprising the steps:
(a) incubating (S)-2-[4-(3-Fluoro-benzyloxy)-benzylamino]-propionamide mesylate anhydrate crystalline modification A1 at ≤ 0° C for one or more milliseconds or seconds or minutes or hours or days, or
(b) incubating (S)-2-[4-(3-Fluoro-benzyloxy)-benzylamino]-propionamide mesylate anhydrate crystalline modification A1 as component of a mixture comprising at least one additional component at ≤ 0° C for one or more milliseconds or seconds or minutes or hours or days.

### Brief description of the drawings

**Figure 1** depicts the powder X-ray diffractogram using Cu-Kα₁ radiation of crystalline modification A1 of (S)-2-[4-(3-Fluoro-benzyloxy)-benzylamino]-propionamide mesylate anhydrate.
**Figure 2** depicts the crystal structure of modification A1 of (S)-2-[4-(3-Fluorobenzyloxy)-benzylamino]-propionamide mesylate anhydrate obtained from powder X-ray diffraction.
**Figure 3** depicts the FT-lR spectrum of crystalline modification A1 of (S)-2-[4-(3-Fluoro-benzyloxy)-benzylamino]-propionamide mesylate anhydrate.
**Figure 4** depicts the FT-Raman spectrum of crystalline modification A1 of (S)-2-[4-(3-Fluoro-benzyloxy)-benzylamino]-propionamide mesylate anhydrate.
**Figure 5** depicts the DSC scan profile of crystalline modification A1 of (S)-2-[4-(3-Fluoro-benzyloxy)-benzylamino]-propionamide mesylate anhydrate.
**Figure 6** depicts the TGA scan profile of crystalline modification A1 of (S)-2-[4-(3-Fluoro-benzyloxy)-benzylamino]-propionamide mesylate anhydrate.
**Figure 7** depicts the powder X-ray diffractogram using Cu-Kα₁ radiation of crystalline modification NF6 of (S)-2-[4-(3-Fluoro-benzyloxy)-benzylamino]-propionamide mesylate anhydrate.
**Figure 8** depicts the variable temperature X-ray diffractogram using Cu-Kα₁ radiation of crystalline modifications NF6 and A1 of (S)-2-[4-(3-Fluoro-benzyloxy)-benzylamino]-propionamide mesylate anhydrate.
**Figure 9** depicts the DSC scan profile of crystalline modifications NF6 and A1 of (S)-2-[4-(3-Fluoro-benzyloxy)-benzylamino]-propionamide mesylate anhydrate.

Even without further details, it is assumed that a person skilled in the art will be able to utilize the above description in the broadest scope. The preferred embodiments should therefore merely be regarded as descriptive disclosure, which is absolutely not limiting in any way.

The contents of all cited references are hereby incorporated by reference in their entirety. The invention is explained in more detail by means of the following examples without, however, being restricted thereto.

### Examples

### Example 1:

### Production of (S)-2-[4-(3-Fluoro-benzyloxy)-benzylamino]-propionamide mesylate anhydrate in its crystalline modification A1

Crystalline modification A1 can be synthesized according to the procedures stated in WO 2007/147491 and WO 2009/074478.

### Example 2:

### Structural and physico-chemical characterization of (S)-2-[4-(3-Fluoro-benzyloxy)-benzylamino]-propionamide mesylate anhydrate in its crystalline modification A1

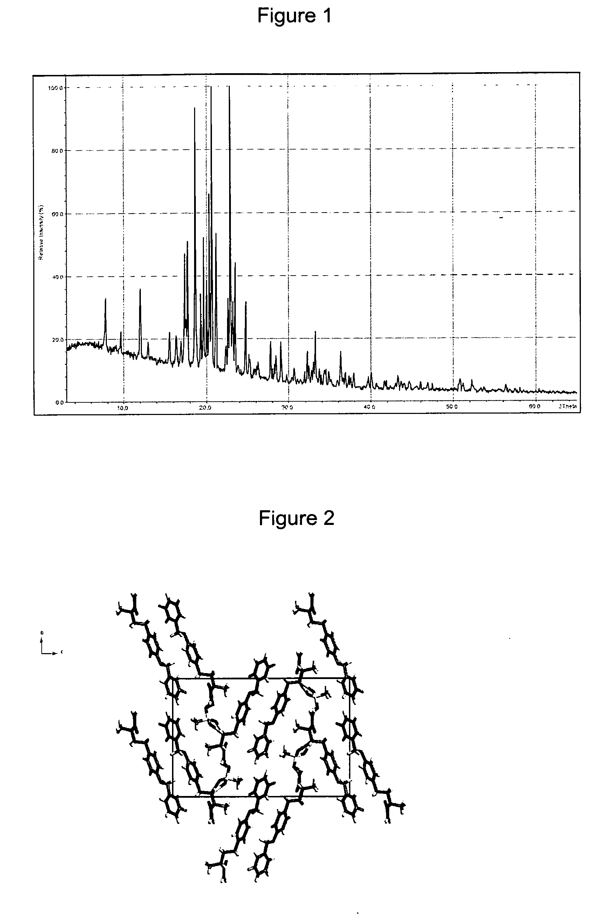

A powder X-Ray Diffraction pattern of crystalline modification A1 was obtained by standard techniques as described in the European Pharmacopeia 6^{th} Edition chapter 2.9.33, and is characterized by the following powder X-ray diffractogram (Cu-Kα₁ radiation, λ = 1.5406 Å, Stoe StadiP 611 KL diffractometer) depicted in **Figure 1****.**

Crystalline modification A1 can be characterized by the following peaks in the X-ray diffractogram. Three lists are given, the first one comprising the most extended listing, the second and third focusing on more important and the most important peaks in the X-ray diffractogram.

Powder X-ray peak list of crystalline modification A1 (2θ values for Cu-K_{α1} radiation):

| No. | 2 θ [º] | D[Å] | h | k | l |
|---|---|---|---|---|---|
| 1 | 7.8 | 11.4 | 0 | 0 | 2 |
| 2 | 9.6 | 9.2 | 0 | 1 | 2 |
| 3 | 12.0 | 7.4 | 0 | 2 | 1 |
| 4 | 13.0 | 6.8 | 0 | 1 | 3 |
| 5 | 15.6 | 5.7 | 0 | 0 | 4 |
| 6 | 17.4 | 5.1 | 1 | 1 | 1 |
| 7 | 17.7 | 5.0 | 1 | 0 | 2 |
| 8 | 18.6 | 4.8 | 1 | 1 | 2 |
| 9 | 19.3 | 4.6 | 0 | 2 | 4 |
| 10 | 20.3 | 4.4 | 0 | 1 | 5 |
| 11 | 20.6 | 4.3 | 1 | 1 | 3 |
| 12 | 22.8 | 3.9 | 1 | 2 | 3 |
| 13 | 29.0 | 3.1 | 1 | 1 | 6 |

Powder X-ray peak list of crystalline modification A1 (2θ values for Cu-K_{α1} radiation), comprising more important peaks:

| No. | 2 θ [°] | D[Å] | h | k | l |
|---|---|---|---|---|---|
| 1 | 7.8 | 11.4 | 0 | 0 | 2 |
| 2 | 12.0 | 7.4 | 0 | 2 | 1 |
| 3 | 15.6 | 5.7 | 0 | 0 | 4 |
| 4 | 17.7 | 5.0 | 1 | 0 | 2 |
| 5 | 18.6 | 4.8 | 1 | 1 | 2 |
| 6 | 19.3 | 4.6 | 0 | 2 | 4 |
| 7 | 20.3 | 4.4 | 0 | 1 | 5 |
| 8 | 20.6 | 4.3 | 1 | 1 | 3 |
| 9 | 22.8 | 3.9 | 1 | 2 | 3 |
| 10 | 29.0 | 3.1 | 1 | 1 | 6 |

Powder X-ray peak list of crystalline modification A1 (2θ values for Cu-K_{α1} radiation), comprising most important peaks:

| No. | 2 θ [°] | D [A] | h | k | l |
|---|---|---|---|---|---|
| 1 | 18.6 | 4.8 | 1 | 1 | 2 |
| 2 | 20.3 | 4.4 | 0 | 1 | 5 |
| 3 | 20.6 | 4.3 | 1 | 1 | 3 |
| 4 | 22.8 | 3.9 | 1 | 2 | 3 |

A powder structure solution has been obtained from a Debye-Scherrer measurement of crystalline modification A1 with Co-K_{α1} radiation using the program DASH 3.1 by CCDC yielding following structure data:

| | |
|---|---|
| Crystal system | orthorhombic |
| Space group | *P*2,2₁2₁, chiral |
| Lattice parameters: | |
| *a* | 5.6 Å |
| *b* | 15.6 Å |
| *c* | 22.8 Å |
| Unit cell volume | 1969 Å³ |

A crystal structure of crystalline modification A1 obtained from powder X-ray data is depicted in **Figure 2****.**

Crystalline modification A1 can be further characterized by infrared and Ramanspectroscopy. FT-Raman and FT-lR spectra have been obtained by standard techniques as described in the European Pharmacopeia 6^{th} Edition chapter 2.02.24 and 2.02.48. For measurement of the FT-lR and FT-Raman-spectra a Bruker Vector 22 and a Bruker RFS 100 spectrometer have been used. FT-lR spectra have been baseline corrected using Bruker OPUS software. FT-Raman spectra have been vector normalized using the same software.

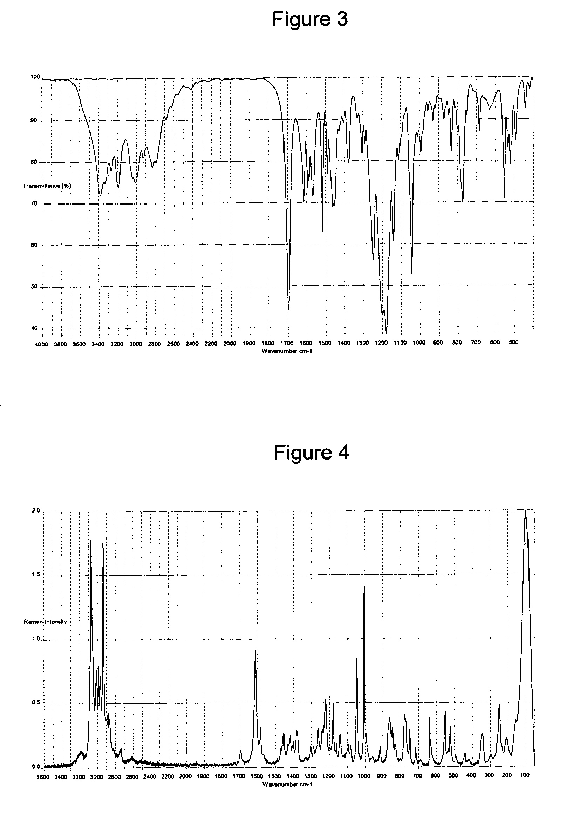

An FT-lR spectrum was obtained using a KBr pellet as sample preparation technique. The FT-lR spectrum is depicted in **Figure 3** and the band positions are given below.

Crystalline modification A1 IR band positions ±2 cm⁻¹ (relative intensity*) 3384 cm⁻¹ (m); 3196 cm⁻¹ (m); 3013 cm⁻¹ (m); 2829 cm⁻¹ (m);1698 cm⁻¹ (s); 1616 cm⁻¹ (m); 1567 cm⁻¹ (m); 1517 cm⁻¹ (m); 1463 cm⁻¹ (m); 1378 cm⁻¹ (m); 1248 cm⁻¹ (m); 1180 cm⁻¹ (s); 1141 cm⁻¹ (m); 1045 cm⁻¹ (s); 834 cm⁻¹ (w); 773 cm⁻¹ (m); 685 cm⁻¹ (w); 553 cm⁻¹ (m); 522 cm⁻¹ (w)

*"s" = strong ,"m" = medium, "w" = weak

An FT-Raman spectrum is depicted in **Figure 4** and the band positions are given below.

Crystalline modification A1 Raman band positions ±2 cm⁻¹ (relative intensity*): 3073 cm⁻¹ (s); 3013 cm⁻¹ (m); 2992 cm⁻¹ (m); 2936 cm⁻¹ (s); 1614 cm⁻¹ (m); 1222 cm⁻¹ (w); 1181 cm⁻¹ (w); 1046 cm⁻¹ (m); 1005 cm⁻¹ (s); 862 cm⁻¹ (w); 780 cm⁻¹ (w); 639 cm⁻¹ (w); 553 cm⁻¹ (w)

*"s" = strong ,"m" = medium, "w" = **weak**

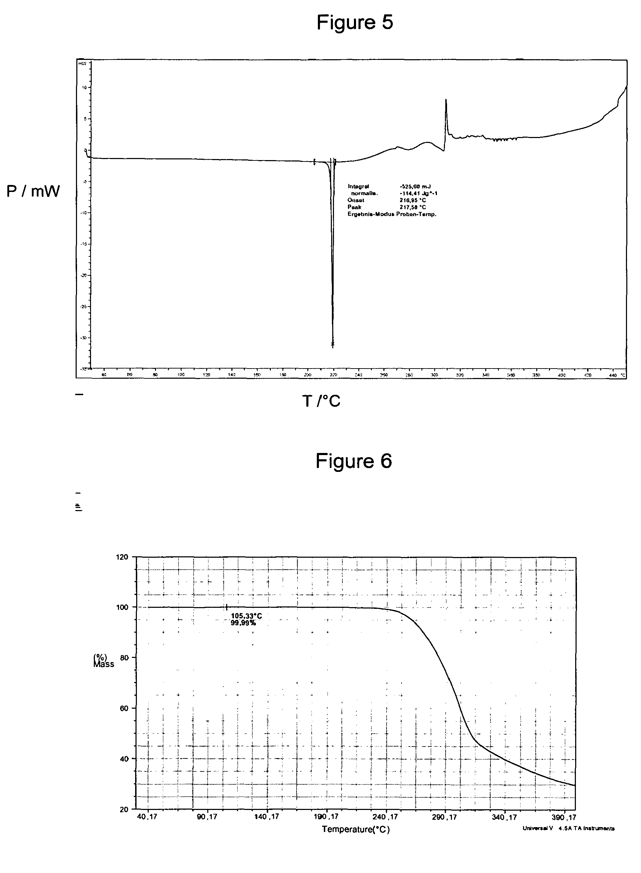

Differential Scanning Calorimetry (DSC) has been performed on Mettler Toledo DSC 821^{e} at 5 K/min starting at room temperature and nitrogen purge gas at 50 mL/min. The DSC scan of crystalline modification A1 depicted in **Figure 5** is not showing enthalpic events till the sharp peak at about 217°C, suggesting melting, and the following decomposition at higher temperatures.

Thermogravimetric Analysis (TGA) was performed on TA-l Q5000 with 5 K/min starting at 40 °C and nitrogen purge gas at 50 mL/min). The TGA scan of crystalline modification A1 depicted in **Figure 6** does not show any insignificant weight-loss until decomposition beginning at about 230°C.

Crystalline Modification A1 can be classified as non-hygroscopic acc. to Ph. Eur. criteria.
Karl Fisher titration has been performed on Mettler AT201 equipped with 756 KF Coulometer attesting crystalline modification A1 a water content of 0.06%, which can be ascribed to physisorbed water on the crystal surface.

### Example 3:

### Production of (S)-2-[4-(3-Fluoro-benzyloxy)-benzylamino]-propionamide mesylate anhydrate in its crystalline modification NF6

Cooling of (S)-2-[4-(3-Fluoro-benzyloxy)-benzylamino]-propionamide mesylate salt crystalline modification A1 below 0 °C yields crystalline modification NF6.

### Example 4:

### Structural and physico-chemical characterization of (S)-2-[4-(3-Fluoro-benzyloxy)-benzylamino]-propionamide mesylate anhydrate in its crystalline modification NF6

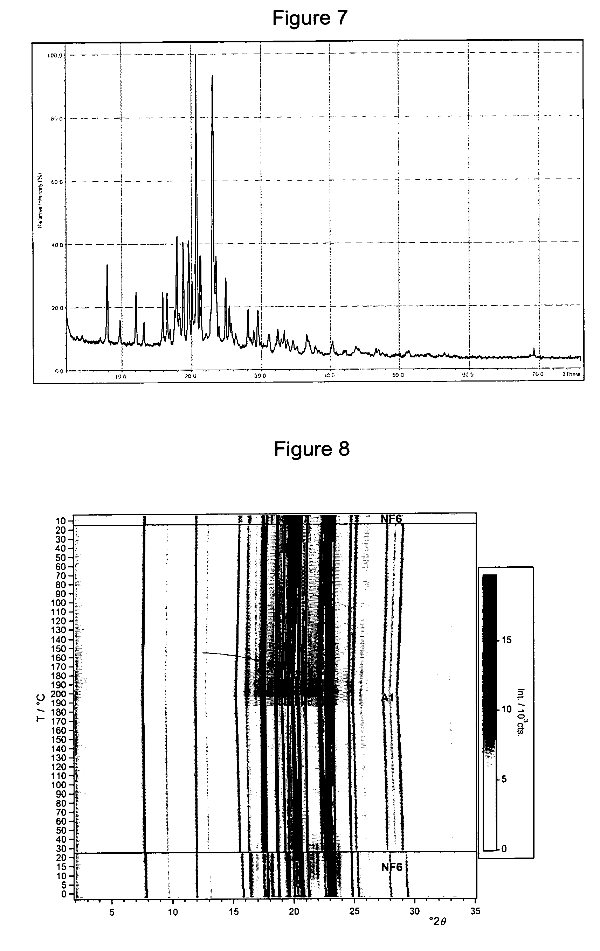

A Powder X-Ray Diffraction pattern of crystalline modification NF6 was obtained by standard techniques as described in the European Pharmacopeia 6^{th} Edition chapter 2.9.33, and is characterized by the following powder X-ray diffractogram (Cu-Kα₁ radiation, λ = 1.5406 Å, Stoe StadiP 611 KL diffractometer) depicted in **Figure 7** (powder X-ray diffractogram of crystalline modification NF6 obtained ≤ 0°C in T-XRD).

The variable temperature powder X-ray diffratogram of crystalline modification NF6 depicted in **Figure 8** originates from a temperature controlled X-ray starting at -35°C heating till 200 °C and cooling down again to -35°C.

Crystalline modification NF6 can be characterized by the following peaks in the powder X-ray diffractogram. Three lists are given, the first one comprising the most extended listing, the second and third focusing on more important and the most important peaks in the powder X-ray diffractogram.

Powder X-ray peak list of crystalline modification NF6 (2θ values for Cu-K_{α1} radiation):

| **Peak No.** | **d/**Å **at -5 °C** | **°2θ at -5 °C (Cu-K**α**₁ radiation) ±0.1°** | **h** | **k** | **l** |
|---|---|---|---|---|---|
| 1 | 20.1 | 4.4 | 0 | 1 | 1 |
| 2 | 11.2 | 7.9 | 0 | 2 | 0 |
| 3 | 7.3 | 12.1 | 0 | 1 | 6 |
| 4 | 5.6 | 15.9 | 0 | 4 | 0 |
| 5 | 5.0 | 17.9 | 1 | 2 | 0 |
| 6 | 4.7 | 18.8 | 1 | 2 | 3 |
| 7 | 4.5 | 19.6 | 0 | 4 | 6 |
| 8 | 4.5 | 19.7 | 1 | 0 | 6 |
| 9 | 4.4 | 20.1 | 1 | 1 | 6 |
| 10 | 4.3 | 20.7 | 0 | 5 | 3 |
| 11 | 4.3 | 20.8 | 1 | 3 | 3 |
| 12 | 4.2 | 21.2 | 1 | 2 | 6 |
| 13 | 3.9 | 23.0 | 0 | 5 | 6 |
| 14 | 3.9 | 23.0 | 1 | 3 | 6 |
| 15 | 3.8 | 23.2 | 0 | 1 | 12 |
| 16 | 3.8 | 23.4 | 0 | 4 | 9 |
| 17 | 3.8 | 23.5 | 1 | 0 | 9 |
| 18 | 3.6 | 24.8 | 1 | 2 | 9 |
| 19 | 3.2 | 28.1 | 1 | 5 | 6 |

Powder X-ray peak list of crystalline modification NF6 (2θ values for Cu-K_{α1} radiation), comprising more important peaks:

| **Peak No.** | **d/Å at -5 °C** | **°2θ at -5 °C (Cu-K**α**₁ radiation) ±0.1°** | **h** | **k** | **l** |
|---|---|---|---|---|---|
| 1 | 11.2 | 7.9 | 0 | 2 | 0 |
| 2 | 5.0 | 17.9 | 1 | 2 | 0 |
| 3 | 4.7 | 18.8 | 1 | 2 | 3 |
| 4 | 4.5 | 19.6 | 0 | 4 | 6 |
| 5 | 4.4 | 20.1 | 1 | 1 | 6 |
| 6 | 4.3 | 20.7 | 0 | 5 | 3 |
| 7 | 4.2 | 21.2 | 1 | 2 | 6 |
| 8 | 3.9 | 23.0 | 1 | 3 | 6 |
| 9 | 3.8 | 23.5 | 1 | 0 | 9 |
| 10 | 3.6 | 24.8 | 1 | 2 | 9 |

Powder X-ray peak list of crystalline modification NF6 (2θ values for Cu-K_{α1} radiation), comprising most important peaks:

| **Peak No.** | **d/Å at -5 °C** | **°2θ at -5 °C (Cu-K**α**₁ radiation) ±0.1º** | **h** | **k** | **l** |
|---|---|---|---|---|---|
| 1 | 5.0 | 17.9 | 1 | 2 | 0 |
| 2 | 4.7 | 18.8 | 1 | 2 | 3 |
| 3 | 4.5 | 19.6 | 0 | 4 | 6 |
| 4 | 4.3 | 20.7 | 0 | 5 | 3 |
| 5 | 3.9 | 23.0 | 1 | 3 | 6 |

Crystalline modification NF6 is further characterized by the following parameter:
orthorhombic, space group P2₁2₁2₁
lattice constants (at -5 °C):
   a = 5.5Å,
   b = 22.3 Å
   c = 46.6 Å
   cell volume = 5771 Å³

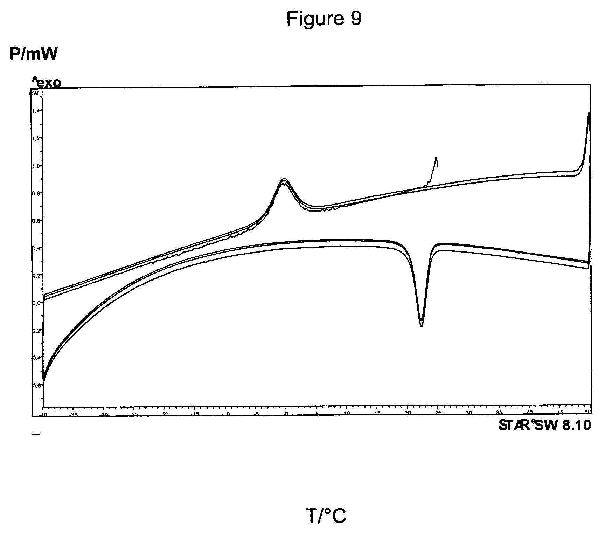

DSC has been performed on Mettler Toledo DSC 821^{e} at 5 K/min and nitrogen purge gas at 50 mUmin. DSC started at 25 °C cooling down to -40 °C and heating to 50 °C before cooling down to -40 °C. This cycle was performed three times (cycle two and three started at 50 °C). The DSC scan of crystalline modification NF6 depicted in **Figure 9** shows an exothermic event while cooling below about 0 °C. When the substance is heated an endothermic event occurs at about 22.5 °C. The occurring transformation is completely reversible.

## Claims

1. (S)-2-[4-(3-Fluoro-benzyloxy)-benzylamino]-propionamide mesylate anhydrate.

2. The compound according to claim 1 in its crystalline modification NF6, which is **characterized by** XRD peaks comprising 17.9°, 18.8°, 19.6°, 20.7° and 23.0° (in °2θ using Cu-Kα₁, radiation, ± 0.1° at -5 °C).

3. The compound according to any of claims 1 to 2, which is **characterized by** powder XRD peaks comprising 7.9°, 17.9°, 18.8°, 19.6°, 20.1 °, 20.7°, 21.2°, 23.0°, 23.5° and 24.8° (in °2θ using Cu-Kα₁ radiation, ± 0.1° at -5 °C).

4. The compound according to any of claims 1 to 3, which is **characterized by** the following XRD data:
**Form NF6:**
| **Peak No.** | **d/Å at -5 °C** | **°2θ at -5 °C (Cu-K**α**₁ radiation ±0.1°)** |
|---|---|---|
| 1 | 20.1 | 4.4 |
| 2 | 11.2 | 7.9 |
| 3 | 7.3 | 12.1 |
| 4 | 5.6 | 15.9 |
| 5 | 5.0 | 17.9 |
| 6 | 4.7 | 18.8 |
| 7 | 4.5 | 19.6 |
| 8 | 4.5 | 19.7 |
| 9 | 4.4 | 20.1 |
| 10 | 4.3 | 20.7 |
| 11 | 4.3 | 20.8 |
| 12 | 4.2 | 21.2 |
| 13 | 3.9 | 23.0 |
| 14 | 3.9 | 23.0 |
| 15 | 3.8 | 23.2 |
| 16 | 3.8 | 23.4 |
| 17 | 3.8 | 23.5 |
| 18 | 3.6 | 24.8 |
| 19 | 3.2 | 28.1 |

5. The compound according to any of claims 1 to 4, which is **characterized by** the following lattice constants (at -5 °C):
a = 5.5 Å
b = 22.3 Å
c = 46.6 Å

6. A pharmaceutical composition comprising a therapeutically effective amount of at least one compound according to any of claims 1 to 5.

7. A pharmaceutical composition according to claim 6 comprising 1% to 50% (S)-2-[4-(3-Fluoro-benzyloxy)-benzylamino]-propionamide mesylate anhydrate in its crystalline modification NF6 according to any of claims 2 to 5 and 1% to 50% (S)-2-[4-(3-Fluoro-benzyloxy)-benzylamino]-propionamide mesylate anhydrate in its crystalline modification A1, which is **characterized by** XRD peaks comprising 18.6°, 20.3°, 20.6° and 22.8° (in °2θ using Cu-Kα₁ radiation, ± 0.1°), preferably **characterized by** XRD peaks comprising 7.8°, 12.0°, 15.6°, 17.7°, 18.6°, 19.3°, 20.3°, 20.6°, 22.8° and 29.0° (in °2θ using Cu-Kα₁ radiation, ± 0.1°), and even more preferably **characterized by** the following XRD data:
**Form A1:**
| **Peak No.** | **d/**Å | **º2**θ **(Cu-K**α**₁ radiation) ±0.1°** |
|---|---|---|
| 1 | 11.4 | 7.8 |
| 2 | 9.2 | 9.6 |
| 3 | 7.4 | 12.0 |
| 4 | 6.8 | 13.0 |
| 5 | 5.7 | 15.6 |
| 6 | 5.1 | 17.4 |
| 7 | 5.0 | 17.7 |
| 8 | 4.8 | 18.6 |
| 9 | 4.6 | 19.3 |
| 10 | 4.4 | 20.3 |
| 11 | 4.3 | 20.6 |
| 12 | 3.9 | 22.8 |
| 13 | 3.1 | 29.0 |

8. The pharmaceutical composition as claimed in any of claims 6 to 7 further comprising at least one additional compound selected from the group consisting of physiologically acceptable excipients, auxiliaries, adjuvants, diluents, carriers and/or additional pharmaceutically active substances other than the compounds according to any of claims 1 to 5.

9. Use of a compound according to any of claims 1 to 5 or a pharmaceutical composition according to any of claims 6 to 8 as a dopamine modulator, monoamine oxidase B inhibitor and/or dopamine reuptake inhibitor.

10. Medicament comprising at least one compound according to any of claims 1 to 5 or a pharmaceutical composition according to any of claims 6 to 8.

11. Medicament according to claim 10 for use in the treatment and/or prophylaxis of physiological and/or pathophysiological conditions selected from the group consisting of: "central nervous system disorders, epilepsy, Parkinson's disease, neurodegenerative processes, depression, spasm, hypnosis, analgesia, chronic pain, neuropathic pain, head pain conditions, migraine, headache, cluster headache, neuralgia, hemicrania, facial pain, arachnoiditis, severe headache, restless legs syndrome, addictive disorders, motor restlessness, creeping, burning, pulling sensations, drug abuse, severe alcoholism, reward deficiency syndrome, cognitive disorders, autism, dyslexia, attention deficit hyperactivity disorder, anxiety, schizophrenia, obsessive compulsive disorders, psychosis, bipolar disorder, Tourette's syndrome, mild cognitive impairment and disorders of learning in children, adolescents and adults, age associated memory impairment, age associated cognitive decline, Alzheimer's disease, Down's Syndrome, traumatic brain injury Huntington's disease, progressive supranuclear Palsy, HIV, stroke, vascular diseases, Pick's or Creutzfeldt-Jacob diseases, multiple sclerosis".

12. The medicament as claimed in any of claims 10 to 11, wherein in such medicament comprises at least one additional pharmacologically active substance.

13. The medicament as claimed in any of claims 10 to 11, wherein the medicament is applied before and/or during and/or after treatment with at least one additional pharmacologically active substance.

14. Kit comprising a therapeutically effective amount of at least one compound according to any of claims 1 to 5 and/or at least one pharmaceutical composition as claimed in any of claims 6 to 8 and a therapeutically effective amount of at least one further pharmacologically active substance other than the compounds as claimed in any of claims 1 to 5.

15. Process for manufacturing crystalline modification NF6 of (S)-2-[4-(3-Fluorobenzyloxy)-benzylamino]-propionamide mesylate anhydrate according to any of claims 2 to 5 comprising the steps:
(a) incubating (S)-2-[4-(3-Fluoro-benzyloxy)-benzylamino]-propionamide mesylate anhydrate crystalline modification A1 at ≤ 0° C for one or more milliseconds or seconds or minutes or hours or days, or
(b) incubating (S)-2-[4-(3-Fluoro-benzyloxy)-benzylamino]-propionamide mesylate anhydrate crystalline modification A1 as component of a mixture comprising at least one additional component at ≤ 0° C for one or more milliseconds or seconds or minutes or hours or days.
